# EUROPÄISCHE PATENTSCHRIFT

(11) **EP 0 632 011 B1**
(45) Veröffentlichungstag und Bekanntmachung des Hinweises auf die Patenterteilung: **15.10.1997**
(21) Anmeldenummer: 94109701.6
(22) Anmeldetag: 23.06.1994
(51) Int. Cl.: C07C 69/708, C07C 67/31

(54) **Verfahren zur Herstellung von Alkoxy-carbonsäureestern**
Process for the preparation of alcoxy carboxylic acid esters
Procédé de préparation d'esters d'acides alcoxycarboxyliques

(30) Priorität: 01.07.1993 DE 4321871
(43) Veröffentlichungstag der Anmeldung: 04.01.1995
(73) Patentinhaber: BASF Aktiengesellschaft, 67063 Ludwigshafen (DE)
(72) Erfinder: Eller, Karsten, Dr., D-67059 Ludwigshafen (DE)

(56) Entgegenhaltungen:
- Keine einschlägigen Dokumente gefunden

## Beschreibung

Die vorliegende Erfindung betrifft ein Verfahren zur Herstellung von Alkoxy-carbonsäureestern durch Umsetzung von Hydroxy-carbonsäureestern mit Alkoholen in Gegenwart von Zeolithen oder hydrothermal hergestellten Phosphaten bei erhöhten Temperaturen.

Unsymmetrische Ether können durch Williamson-Synthese (siehe z.B. J. March, Advanced Organic Chemistry, Wiley, New York, 3rd Ed., 1985, Seite 342 bis 343, oder Kirk-Othmer, Encyclopedia of Chemical Technology, Wiley, New York, 3rd Ed., 1980, Vol. 9, Seite 384 bis 385) hergestellt werden. Diese Synthese besitzt den Nachteil der zweistufigen Reaktionsführung und des Zwangsanfalls von salzartigen Koppelprodukten.

Es ist bekannt, daß saure Katalysatoren wie Al₂O₃ (Wade et al., J. Colloid Interface Sci. 21, 1966, 349 bis 357) für die Bildung symmetrischer Ether verwendet werden können.

So beschreibt z.B. EP 148 626 die Bildung von Dimethylether aus Synthesegas über intermediär gebildetes Methanol an Aluminosilikaten, welche speziell mit Stickstoffverbindungen vorbehandelt wurden; die genannten Umsätze sind allerdings gering.

Aus der EP-A-340 324 ist eine technische Synthese von Dimethylether aus Methanol an gamma-Al₂O₃-Katalysatoren mit geringen Mengen SiO₂ (< 1 %) bekannt.

Aus der GB-A-2 164 636 und der GB-A-2 179 563 ist die Verwendung synthetischer Beidellit-Smectite oder modifizierter Montmorillonite für symmetrische Veretherungen von n-Pentanol zum 1,1-Dipentylether bekannt.

Aus der EP-A-31 252 ist die Verwendung von Bentoniten bekannt, die neben den gewünschten symmetrischen Ethern auch gleichzeitig erhebliche Mengen Alkene durch Dehydratisierung der Alkohole liefert.

Der vorliegenden Erfindung lag daher die Aufgabe zugrunde, den oben genannten Nachteilen abzuhelfen.

Demgemäß wurde ein neues und verbessertes Verfahren zur Herstellung von Alkoxy-carbonsäureestern der allgemeinen Formel I 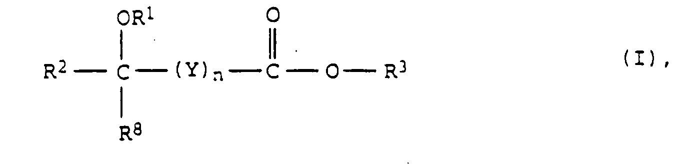 in der
- Y: CR⁴R⁵, CR⁴R⁵-CR⁶R⁷
- R¹,R²,R³,R⁴,R⁵,R⁶,R⁷,R⁸: C₁- bis C₂₀-Alkyl, C₃- bis C₈-Cycloalkyl, Aryl, C₇- bis C₂₀-Aralkyl oder C₇- bis C₂₀-Alkylaryl und
- R²,R⁴,R⁵,R⁶,R⁷,R⁸: Wasserstoff,
- n: 0 oder 1
bedeuten, durch Umsetzung von Hydroxy-carbonsäureestern der allgemeinen Formel II 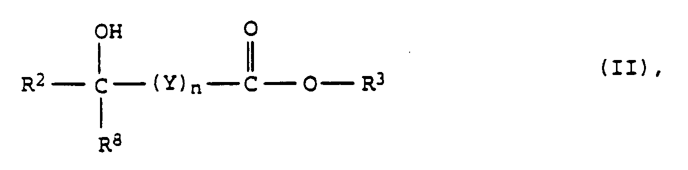 in der R², R³, R⁸, Y und n die oben genannten Bedeutungen haben, mit Alkoholen der allgemeinen Formel III

R¹-OH (III),

in der R¹ die oben genannten Bedeutungen hat, in Gegenwart von Heterogenkatalysatoren bei Temperaturen von 100 bis 400°C und Drücken von 0,01 bis 150 bar gefunden, welches dadurch gekennzeichnet ist, daß man als Heterogenkatalysatoren Zeolithe und/oder hydrothermal hergestellte Phosphate einsetzt.

Das erfindungsgemäße Verfahren kann wie folgt durchgeführt werden:

Die Edukte, Hydroxy-carbonsäureester II und Alkohol III, können entweder gemischt oder einzeln, flüssig oder bevorzugt gasförmig einem mit einem Heterogenkatalysator gefüllten Reaktor, bevorzugt an Festbettkontakten in Linear- oder Wendelreaktoren, beispielsweise mittels eines Vorverdampfers, zugeführt werden, und bei Temperaturen von 100 bis 400°C, bevorzugt 150 bis 350°C, besonders bevorzugt 180 bis 320°C, und Drücken von 0,01 bis 150 bar, bevorzugt 0,1 bis 5 bar, besonders bevorzugt bei Normaldruck (Atmosphärendruck), umgesetzt werden. Die Reaktionstemperatur kann hierbei bevorzugt so gewählt werden, daß sie oberhalb des Verdampfungspunktes der am schwersten flüchtigsten Komponente liegt. Zu hohe Temperaturen begünstigen die Bildung von symmetrischen Ethern.

Zweckmäßigerweise geht man von einer vorgefertigten Mischung aus, die über eine Pumpe einem Vorratsbehälter entnommen wird. Die Reaktion kann in Gegenwart eines inerten Trägergasstromes, wie Stickstoff, Kohlendioxid oder Argon, durchgeführt werden.

Bei einer bevorzugten Ausführungsform des erfindungsgemäßen Verfahrens wird die Umsetzung mit 10 bis 75 Gew.-% igen alkoholischen Lösungen von Hydroxycarbonsäureestern II durchgeführt. Als Alkohole eignen sich dafür C₁- bis C₂₀-Alkanole, bevorzugt C₁- bis C₈-Alkanole, besonders bevorzugt C₁- bis C₄-Alkanole wie Methanol, Ethanol, n-Propanol, iso-Propanol, n-Butanol, iso-Butanol, sec.-Butanol und tert.-Butanol.

Die Bildung der Hydroxycarbonsäureester ist bevorzugt in situ möglich, indem man eine Mischung aus Säure und Alkohol einsetzt, die unter den Reaktionsbedingungen am Katalysator verestert wird. In dieser Ausführung der Erfindung ist es folglich möglich, in einem Schritt von der Hydroxycarbonsäure zum Alkoxycarbonsäureester zu gelangen.

Die Heterogenkatalysatoren können entweder vor der Einfüllung in den Reaktor getrocknet werden, oder die Trocknung erfolgt vor der Reaktion direkt im Reaktor. Vorteilhaft werden hierzu Temperaturen verwendet, die über dem Siedepunkt des Wassers liegen und unterhalb der Umwandlungs- oder Schmelztemperatur der Molekularsiebkristalle, besonders vorteilhaft sind Temperaturen zwischen 300 und 600°C.

Desaktivierte Heterogenkatalysatoren können durch Abbrennen des abgelagerten Kokses regeneriert werden. Die Regenerierung kann hierbei außerhalb oder direkt im Reaktor stattfinden. In Gegenwart von Luft oder Sauerstoff erfolgt die Regenerierung nachhaltiger und gründlicher. Als vorteilhaft hat sich das Überleiten von Luft im Reaktor bei Temperaturen zwischen 300 und 600°C erwiesen.

Als Heterogenkatalysatoren eignen sich Zeolithe, bevorzugt Zeolithe aus der Mordenit-Gruppe, Zeolithe mittlerer Porengröße wie die des Pentasil-Typs ZSM-5, engporige Zeolithe vom Erionit- bzw. Chabasit-Typ, weitporige Zeolithe vom Faujasit-Typ, z.B. X- oder Y- Zeolithe, oder L-Zeolithe. In die letzte Gruppe gehören auch die sogenannten "ultrastabilen" Zeolithe des Faujasittyps, d.h. dealuminierte Zeolithe. Ebenfalls können Phosphate mit Zeolithstruktur eingesetzt werden, d.h. Alumophosphate (AlPOs) wie AlPO-5, AlPO-9, AlPO-11, AlPO-12, AlPO-14, etc. oder Silicoalumophosphate (SAPOs) wie SAPO-5, SAPO-11, SAPO-31 oder SAPO-34.

Besonders bevorzugt sind X-Zeolithe in der Alkali- oder Erdalkaliform oder hydrothermal hergestellte Phosphate, bevorzugt Alumophosphate (AlPO's) oder Silicoalumophosphate (SAPO's).

Unter Zeolithen versteht man dabei kristalline Aluminosilikate, die eine hochgeordnete Struktur mit einem dreidimensionalen Netzwerk von SiO₄- und AlO₄-Tetraedern besitzen, die durch gemeinsame Sauerstoffatome verbunden sind. Das Verhaltnis der Si- und Al-Atome zu Sauerstoff beträgt 1 : 2 (s. Ullmanns Encyklopädie der technischen Chemie, Verlag Chemie, Weinheim, 4. Aufl., 1983, Bd. 24, S. 575). Die Elektrovalenz der Aluminium enthaltenden Tetraeder ist durch Einschluß von Kationen in den Kristall, z.B. Alkali- oder Wasserstoffionen, ausgeglichen. Ein Kationenaustausch ist möglich. Die Räume zwischen den Tetraedern sind vor der Dehydratisierung durch Trocknen bzw. Calcinieren von Wassermolekülen besetzt.

Die Zeolithe werden zumeist in der aziden H-Form oder neutralen Alkaliform angewendet. In den Zeolithen können anstelle von Aluminium auch andere Elemente, wie B, Ga, Fe, Cr, V, As, Sb, Bi, Be oder deren Gemische in das Gitter eingebaut werden, oder das Silizium kann durch ein anderes vierwertiges Element, wie Ge, Ti, Zr, Hf, ersetzt werden.

Entsprechend ihrer Struktur werden Zeolithe in verschiedene Gruppen unterteilt. Eine Zusammenstellung solcher Strukturen wird in der Literatur gegeben (W.M. Meier und D.H. Olson, Atlas of Zeolite Structure Types, Butterworths, London, 2nd Ed., 1987).

Verfahren zur Herstellung von Zeolithen sind mannigfaltig beschrieben. Als Alumophosphate werden für das erfindungsgemäße Verfahren insbesondere unter hydrothermalen Bedingungen synthetisierte Alumophosphate eingesetzt. Beispielsweise wird AlPO-5 synthetisiert, indem man Orthophosphorsäure und Pseudoböhmit (Captal SB®) in Wasser homogen mischt, zu dieser Mischung Tetrapropylammoniumhydroxid gibt und danach bei etwa 150°C 20 bis 60 h unter autogenem Druck in einem Autoklaven umsetzt. Der abfiltrierte AlPO-5 wird getrocknet bei 100 bis 160°C und kalziniert bei 450 bis 550°C. SAPO-5 wird beispielsweise erhalten durch Mischen von SiO₂, suspendiert in wäßriger Tetrapropylammoniumhydroxid-Lösung, mit einer wäßrigen Suspension aus Pseudoböhmit und Orthophosphorsäure und anschließender Umsetzung bei 150 bis 200°C, während 20 bis 200 h unter autogenem Druck, in einem Rührautoklaven. Die Trocknung des abfiltrierten Pulvers erfolgt bei 110 bis 160°C und die Kalzinierung bei 450 bis 550°C.

Die Substituenten Y, R¹, R², R³, R⁴, R⁵, R⁶, R⁷, R⁸ und der Index n haben folgende Bedeutungen:
- Y: - CR⁴R⁵ oder CR⁴R⁵-CR⁶R⁷,
- R¹,R²,R³,R⁴,R⁵,R⁶,R⁷,R⁸: - C₁- bis C₂₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl.
- C₃- bis C₈-Cycloalkyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl und
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl.
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- R²,R⁴,R⁵,R⁶,R⁷,R⁸: zusätzlich Wasserstoff,
- n: 0 oder 1, bevorzugt 0.

### Beispiele

Die Analyse der entstehenden Produkte erfolgte mit gaschromatographischen Methoden, und die Identität der Produkte wurde durch Vergleich mit authentischem Material oder durch Gaschromatographie mit massenspektometrischer Koppelung (GC-MS) sichergestellt.

### Beispiel 1

In einen Wendelreaktor von 0,9 cm Durchmesser und 100 cm Länge werden 10 g X-Zeolith (Fa. Wako, 1/8' oder 1/16' Stränge, versplittet) eingefüllt, welcher über Nacht bei 150°C im Trockenschrank vorbehandelt wurde. Der Reaktor wird auf 240°C aufgeheizt und über eine Pumpe eine Mischung aus 25 Gewichtsteilen Milchsäuremethylester und 75 Gewichtsteilen Methanol mit 10 g h⁻¹ zugegeben. In einem nachgeschalteten Kühler mit Vorlage wird die Produktmischung aufgefangen und in regelmäßigen Abständen Proben zur Analyse entnommen. Nach 4 h Reaktionszeit betrug der Umsatz an Milchsäuremethylester 94 %, die Ausbeute an 2-Methoxypropionsäuremethylester 77,6 % und nach 8 h 92,4 % bzw. 77,2 %.

### Beispiel 2

Analog zu Beispiel 1 wird mit 10 g zeolithanalogem Alumophosphat AlPO-5 bei 300°C Reaktionstemperatur nach 4 h ein Umsatz von 72 % sowie 46,9 % Ausbeute an 2-Methoxypropionsäuremethylester beobachtet.

### Beispiel 3

Analog zu Beispiel 1 wird mit 10 g zeolithanalogem Silicoalumophosphat SAPO-5 bei 300°C Reaktionstemperatur nach 6 h ein Umsatz von 91,2 % sowie 27,5 % Ausbeute an 2-Methoxypropionsäuremethylester beobachtet.

### Beispiel 4

Analog zu Beispiel 1 wird mit 10 g CaX (Molsieb 10 Å) bei 300°C Reaktionstemperatur nach 4 h ein Umsatz von 82 % sowie 45,8 % Ausbeute an 2-Methoxypropionsäuremethylester beobachtet.

### Beispiel 5

In einen Wendelreaktor von 0,9 cm Durchmesser und 100 cm Länge werden 10 g X-Zeolith (Fa. Wako, 1/8' oder 1/16' Stränge, versplittet) eingefüllt, welcher über Nacht bei 150°C im Trockenschrank vorbehandelt wurde. Der Reaktor wird auf 260°C aufgeheizt und über eine Pumpe eine Mischung aus 25 Gewichtsteilen Hydroxyessigsäuremethylester und 75 Gewichtsteilen Methanol mit 10 g h⁻¹ zugegeben. In einem nachgeschalteten Kühler mit Vorlage wird die Produktmischung aufgefangen und in regelmäßigen Abständen Proben zur Analyse entnommen. Nach 4 h Reaktionszeit betrug der Umsatz an Hydroxyessigsäuremethylester 91,6 %, die Ausbeute an Methoxyessigsäure-methylester 90,7 %.

### Beispiel 6

In einen Wendelreaktor von 0,9 cm Durchmesser und 100 cm Länge werden 10 g X-Zeolith (Fa. Wako, 1/8' oder 1/16' Stränge, versplittet) eingefüllt, welcher über Nacht bei 150°C im Trockenschrank vorbehandelt wurde. Der Reaktor wird auf 240°C aufgeheizt und über eine Pumpe eine Mischung aus 25 Gewichtsteilen 2-Hydroxyhexansäureethylester und 75 Gewichtsteilen Methanol mit 10 g h⁻¹ zugegeben. In einem nachgeschalteten Kühler mit Vorlage wird die Produktmischung aufgefangen und in regelmäßigen Abständen Proben zur Analyse entnommen. Nach 5 h Reaktionszeit betrug der Umsatz an 2-Hydroxyhexansäureethylester 73,2 % und die Ausbeute an 2-Methoxyhexansäure-methylester 41,2 %.

### Beispiel 7

In einen Linearreaktor von 0,9 cm Durchmesser und 22,5 cm Länge werden 10 g X-Zeolith (Fa. Wako, 1/8' oder 1/16' Stränge) eingefüllt und über Nacht im Reaktor bei 260°C getrocknet. Über eine Pumpe wird bei derselben Temperatur eine Mischung aus 25 Gewichtsteilen Milchsäuremethylester und 75 Gewichtsteilen Methanol mit 8 g h⁻¹ zugegeben. In einem nachgeschalteten Kühler mit Vorlage wird die Produktmischung aufgefangen und in regelmäßigen Abständen Proben zur Analyse entnommen. Nach 24 h Reaktionszeit betrug der Umsatz 90,8 % und die Ausbeute an 2-Methoxypropionsäuremethylester 80,7 %.

### Beispiel 8

In einen Linearreaktor von 0,9 cm Durchmesser und 22,5 cm Länge werden 10 g X-Zeolith (Fa. Wako, 1/8' oder 1/16' Stränge, versplittet) eingefüllt, welcher über Nacht bei 150°C im Trockenschrank vorbehandelt wurde. Der Reaktor wird auf 300°C aufgeheizt und über eine Pumpe eine Mischung aus 25 Gewichtsteilen Milchsäuremethylester und 75 Gewichtsteilen Methanol mit 10 g h⁻¹ zugegeben. Durch ein Regelventil wird der Ausgang des Reaktors erst bei 80 bar freigegeben. Nach 7 h betrug der Umsatz 86,8 %, die Ausbeute an 2-Methoxypropionsäuremethylester 65,6 %.

### Beispiel 9

In einen Wendelreaktor von 0,9 cm Durchmesser und 100 cm Länge werden 10 g X-Zeolith (Fa. Wako, 1/8' oder 1/16' Stränge, versplittet) eingefüllt, welcher über Nacht bei 150°C im Trockenschrank vorbehandelt wurde. Der Reaktor wird auf 240°C aufgeheizt und über eine Pumpe eine Mischung aus 25 Gewichtsteilen Milchsäuremethylester und 75 Gewichtsteilen Ethanol mit 10 g h⁻¹ zugegeben. In einem nachgeschalteten Kühler mit Vorlage wird die Produktmischung aufgefangen und in regelmäßigen Abständen Proben zur Analyse entnommen. Nach 1 h Reaktionszeit betrug der Umsatz an Milchsäuremethylester 100 %, die Ausbeute an 2-Methoxypropionsäuremethylester 1,4 %, die Ausbeute an 2-Methoxypropionsäureethylester 2,2 %, die Ausbeute an 2-Ethoxypropionsäure-methylester 6,0 %, und die Ausbeute an 2-Ethoxypropionsäureethylester 13,6 %. Die Ausbeute des Umesterungsproduktes Milchsäureethylester betrug 2,1 %.

## Patentansprüche

1. Verfahren zur Herstellung von Alkoxy-carbonsäureestern der allgemeinen Formel I 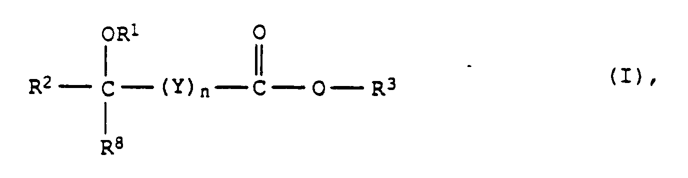 in der
Y CR⁴R⁵, CR⁴R⁵-CR⁶R⁷
R¹,R²,R³,R⁴,R⁵,R⁶,R⁷,R⁸ C₁- bis C₂₀-Alkyl, C₃- bis C₈-Cycloalkyl, Aryl, C₇- bis C₂₀-Aralkyl oder C₇- bis C₂₀-Alkylaryl und
R²,R⁴,R⁵,R⁶,R⁷,R⁸ Wasserstoff,
n 0 oder 1
bedeuten, durch Umsetzung von Hydroxy-carbonsäureestern der allgemeinen Formel II 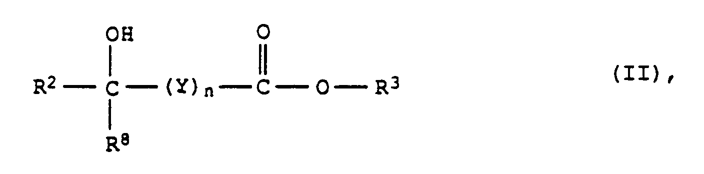 in der R², R³, R⁸, Y und n die oben genannten Bedeutungen haben, mit Alkoholen der allgemeinen Formel III
R¹-OH (III),
in der R¹ die oben genannten Bedeutungen hat, in Gegenwart von Heterogenkatalysatoren bei Temperaturen von 100 bis 400°C und Drücken von 0,01 bis 150 bar, dadurch gekennzeichnet, daß man als Heterogenkatalysatoren Zeolithe und/oder hydrothermal hergestellte Phosphate einsetzt.

2. Verfahren zur Herstellung von Alkoxy-carbonsäureestern I nach Anspruch 1, dadurch gekennzeichnet, daß n = 0 ist.

3. Verfahren zur Herstellung von Alkoxy-carbonsäureestern I nach Anspruch 1, dadurch gekennzeichnet, daß R² Wasserstoff oder Methyl, R⁸ Wasserstoff und n = 0 bedeutet.

4. Verfahren zur Herstellung von Alkoxy-carbonsäureestern I nach Anspruch 1, dadurch gekennzeichnet, daß R¹ für Methyl steht.

5. Verfahren zur Herstellung von Alkoxy-carbonsäureestern I nach Anspruch 1, dadurch gekennzeichnet, daß man als Zeolithe X- oder Y-Zeolithe in der Alkali- oder Erdalkaliform einsetzt.

6. Verfahren zur Herstellung von Alkoxy-carbonsäureestern I nach Anspruch 1, dadurch gekennzeichnet, daß man als hydrothermal hergestellte Phosphate Alumophosphate oder Silicoalumophosphate einsetzt.

7. Verfahren zur Herstellung von Alkoxy-carbonsäureestern I nach Anspruch 1, dadurch gekennzeichnet, daß man die Umsetzung mit 10 bis 75 Gew.-% igen alkoholischen Lösungen von Hydroxycarbonsäureestern durchführt.

## Claims

1. A process for preparing alkoxy carboxylic esters of the general formula I 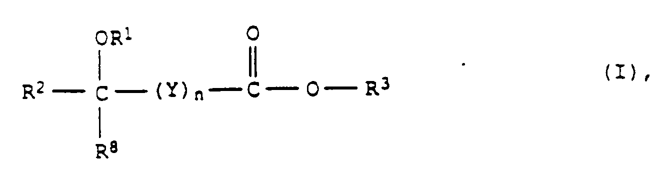 where
Y is CR⁴R⁵, CR⁴R⁵-CR⁶R⁷,
R¹,R²,R³,R⁴,R⁵,R⁶,R⁷,R⁸ are C₁-C₂₀-alkyl, C₃-C₈-cycloalkyl, aryl, C₇-C₂₀-aralkyl or C₇-C₂₀-alkylaryl and
R²,R⁴,R⁵,R⁶,R⁷,R⁸ are hydrogen,
n is 0 or 1
by reacting hydroxycarboxylic esters of the general formula II 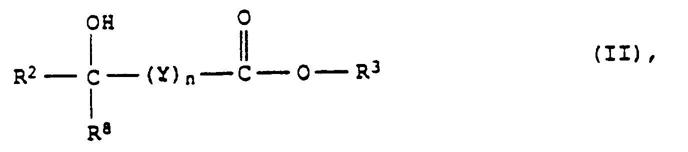 where R², R³, R⁸, Y and n have the abovementioned meanings, with alcohols of the general formula III
R¹-OH (III)
where R¹ has the abovementioned meanings, in the presence of heterogeneous catalysts at from 100 to 400°C under from 0.01 to 150 bar, wherein zeolites and/or hydrothermally produced phosphates are employed as heterogeneous catalysts.

2. A process for preparing alkoxy carboxylic eaters I as claimed in claim 1, wherein n is 0.

3. A process for preparing alkoxy carboxylic eaters I as claimed in claim 1, wherein R² is hydrogen or methyl, R⁸ is hydrogen and n is 0.

4. A process for preparing alkoxy carboxylic esters I as claimed in claim 1, wherein R¹ is methyl.

5. A process for preparing alkoxy carboxylic esters I as claimed in claim 1, wherein X- or Y-zeolites in the alkali metal or alkaline earth metal form are employed as zeolites.

6. A process for preparing alkoxy carboxylic esters I as claimed in claim 1, wherein aluminophosphates or silicoaluminophosphates are employed as hydrothermally produced phosphates.

7. A process for preparing alkoxy carboxylic esters I as claimed in claim 1, wherein the reaction is carried out with from 10 to 75% by weight alcoholic solutions of hydroxy carboxylic esters.

## Revendications

1. Procédé de préparation d'esters d'acides alcoxycarboxyliques de formule générale I 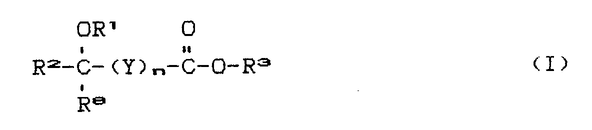 dans laquelle
Y représente CR⁴R⁵, CR⁴R⁵-CR⁶R⁷,
R¹,R²,R³,R⁴,R⁵,R⁶,R⁷,R⁸ représentent des restes alkyle en C₁-C₂₀, cycloalkyle en C₃-C₈, aryle, aralkyle en C₇-C₂₀ ou alkylaryle en C₇-C₂₀, ou
R²,R⁴,R⁵,R⁶,R⁷,R⁸ représentent des atomes d'hydrogène
n est mis pour 0 ou 1,
par réaction d'esters d'acides hydroxycarboxyliques de formule générale II 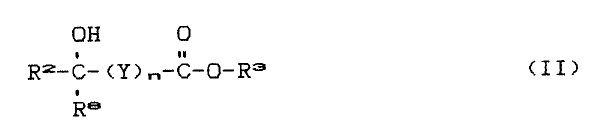 dans laquelle R², R³, R⁸, Y et n ont les significations données ci-dessus, avec des alcools de formule générale III
R¹-OH (III)
dans laquelle R¹ a la signification donnée ci-dessus, en présence de catalyseurs hétérogènes, à des températures de 100 à 400°C et sous des pressions de 0,01 à 150 bar, caractérisé en ce que l'on utilise, comme catalyseurs hétérogènes, des zéolithes et/ou des phosphates préparés par voie hydrothermale.

2. Procédé de préparation d'esters d'acides alcoxycarboxyliques I selon la revendication 1, caractérisé en ce que n = 0.

3. Procédé de préparation d'esters d'acides alcoxycarboxyliques I selon la revendication 1, caractérisé en ce que R² représente un atome d'hydrogène ou un reste méthyle, R⁸ représente un atome d'hydrogène et n = 0.

4. Procédé de préparation d'esters d'acides alcoxycarboxyliques I selon la revendication 1, caractérisé en ce que R¹ est mis pour un reste méthyle.

5. Procédé de préparation d'esters d'acides alcoxycarboxyliques I selon la revendication 1, caractérisé en ce que l'on utilise, comme zéolithes, des zéolithes X ou Y sous la forme alcaline ou alcalino-terreuse.

6. Procédé de préparation d'esters d'acides alcoxycarboxyliques I selon la revendication 1, caractérisé en ce que l'on utilise, comme phosphates préparés par voie hydrothermale, des alumophosphates ou des silicoalumophosphates.

7. Procédé de préparation d'esters d'acides alcoxycarboxyliques I selon la revendication 1, caractérisé en ce que l'on conduit la réaction avec des solutions alcooliques à 10-75% en poids d'esters d'acides hydroxycarboxyliques.
